(19) Europäisches Patentamt
European Patent Office
Office européen des brevets

(11) Publication number: 0 234 481
A1

(12) # EUROPEAN PATENT APPLICATION

(21) Application number: 87102214.1

(22) Date of filing: 17.02.87

(51) Int. Cl.³: A 61 K 45/06
A 61 K 31/65, A 61 K 31/557
//(A61K31/65, 31:557)

(30) Priority: 18.02.86 US 830389

(43) Date of publication of application:
02.09.87 Bulletin 87/36

(84) Designated Contracting States:
AT BE CH DE ES FR GB GR IT LI LU NL SE

(71) Applicant: SYNTEX (U.S.A.) INC.
3401 Hillview Avenue
Palo Alto, California 94304(US)

(72) Inventor: Herschler, Richard C.
1248 Stanwirth Court
Los Altos California 94022(US)

(74) Representative: Barz, Peter, Dr. et al,
Patentanwälte Dipl.-Ing. G. Dannenberg Dr. P. Weinhold,
Dr. D. Gudel Dipl.-Ing. S. Schubert, Dr. P. Barz
Siegfriedstrasse 8
D-8000 München 40(DE)

(54) **Anti-infective injectable formulations.**

(57) Infection associated with subcutaneous or intramuscular injections, especially of $F_2$ α-type prostaglandins, is prevented by novel compositions comprising systemically subtherapeutic amounts of an antibiotic.

EP 0 234 481 A1

# ANTI-INFECTIVE INJECTABLE FORMULATIONS

This invention relates to methods for preventing infection associated with subcutaneous or intramuscular injection of mammals with parenteral formulations, especially prostaglandin formulations, and new compositions containing parenterally administrated compounds with a systemically sub-therapeutic amount of an antibiotic.

## Related Disclosure

It is frequently desirable to administer compounds to livestock by parenteral injection. For example, vaccines, hormones, vitamins and nutritional supplements are frequently administered by intramuscular injection.

$PGF_2\alpha$ and certain $PGF_2\alpha$ derivatives are useful for controlling the reproductive cycles of female mammals. For example, methyl (±)-9α,11α,15α-trihydroxy-16-phenoxy-17,18,19,20-tetranorprosta-4,5,13-(E)-trienoate (known generically as fenprostalene) is used to induce estrus, abortion, or parturition in female mammals, particularly horses, cattle, and swine. Fenprostalene is described in U.S. Pat. No. 3,985,791,

which is incorporated herein by reference in its entirety. Other known $PGF_2\alpha$-type compounds include Cloprostenol, Dinoprost, Luprostiol, Alfaprostol and the like. $PGF_2\alpha$ and its derivatives are most commonly administered via subcutaneous or intramuscular injection.

However, due to the conditions under which livestock are usually injected, this method of administration carries some risk of infection. Administration is most commonly performed in areas which are far from aseptic, and parenteral injection can carry normally harmless bacteria through the hide where it may cause serious infection.

It has now been discovered that one may prevent such infection by incorporating a small amount of an antibiotic in the injection formulation. Surprisingly, the amount of antibiotic required is far less than the usual systemic dosage.

## DEFINITIONS

The term "antibiotic" as used herein includes all commonly used bacteristatic and bactericidal antibiotics, which are suitable for parenteral injection. Antibiotics include aminoglycosides, such as amikacin, gentamicin, kanamycin, neomycin, streptomycin, and tobramycin; cephalosporins, such as cefamandole, cefazolin, cephalexin, cephaloglycin, cephaloridine, cephalothin, cephapirin, and cephradine; macrolides, such as erythromycin and troleandomycin; penicillins, such as penicillin G, amoxicillin, ampicillin, carbenicillin, cloxacillin, dicloxacillin, methicillin, nafcillin, oxacillin, phenethicillin, and ticarcillin; polypeptide antibiotics, such as bacitracin, colistimethate, colistin, polymyxin B; tetracyclines, such as chlortetracycline, demeclocycline, doxycycline, methacycline, minocycline, tetracycline, and

oxytetracycline; and miscellaneous antibiotics such as chloramphenicol, clindamycin, cycloserine, lincomycin, rifampin, spectinomycin, vancomycin, and viomycin. Additional antibiotics are described in "Remington's Pharmaceutical Sciences," 16th Ed., (Mack Pub. Co., 1980), pp. 1121-1178. Presently preferred antibiotics are penicillin, tetracycline, and oxytetracycline, particularly oxytetracycline. Recommended daily systemic doses of oxytetracycline for intramuscular injection in animals range from about 6.5 mg/Kg to about 20 mg/Kg.

The term "parenterally suitable compound" refers to compounds which are commonly administered by subcutaneous or intramuscular injection. Parenterally suitable compounds include, without limitation, vaccines, hormones, vitamins, nutritional supplements, and the like. Preferred parenterally suitable compounds are steroid hormones and prostaglandin derivatives, especially $PGF_2\alpha$ and its derivatives.

The term "$PGF_2\alpha$ derivative" refers to prostaglandin $F_2\alpha$ and prostaglandin derivatives with activity similar to prostaglandin $F_2\alpha$ (also known as dinoprost tromethamine). Presently preferred $PGF_2\alpha$ derivatives are compounds of formula 1:

(1) 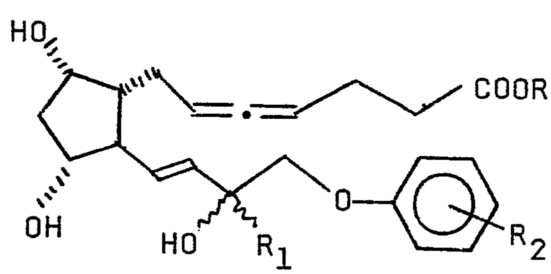

or a pharmaceutically acceptable salt thereof, wherein

R is hydrogen or lower alkyl;

$R_1$ is hydrogen, methyl, or ethyl;

$R_2$ is hydrogen, halo, trifluoromethyl, lower alkyl or lower alkoxy.

The term "lower alkyl" refers to straight or branched saturated monovalent hydrocarbon radicals containing four carbon atoms or less, for example, methyl, ethyl, n-propyl, isopropyl, n-butyl, s-butyl, i-butyl, and t-butyl.

The term "lower alkoxy" refers to radicals of the form RO-, where R is lower alkyl as defined above.

The term "halo" refers to the halogen radicals fluoro, chloro, bromo, or iodo.

The term "effective amount" as used herein refers to the amount of $PGF_2\alpha$ derivative needed to effect induction of estrus, abortion, or parturition in a female mammal. As $PGF_2\alpha$ derivatives are known and used in the art, the effective amount of any particular $PGF_2\alpha$ derivative will be known or readily determined by the practitioner of ordinary skill. In general terms, an effective amount of fenprostalene for induction of parturition, for induction of estrus, or for induction of abortion is from about 0.0022 mg/Kg to about 0.011 mg/Kg, preferably from about 0.0022 mg/Kg to about 0.0044 mg/Kg, and most preferably about 0.0033 mg/Kg. The exact dosage may vary with the species of mammal and the condition being treated. However, such variations are readily predicted by one of ordinary skill.

The term "systemically sub-therapeutic dose" refers to the amount of antibiotic needed to prevent infection associated with intramuscular injection. A systemically sub-therapeutic dose is less than the usual dose prescribed for systemic treatment of infection. For example, oxytetracycline is normally prescribed for mammals in doses of about 11 mg/Kg per day (i.m.), whereas the dosage administered in the practice of the invention can be less than about 0.20 mg/Kg per

injection. A systemically sub-therapeutic amount of oxytetracycline for prevention of infection associated with intramuscular injection ranges from about 0.018 mg/Kg to about 0.14 mg/Kg, preferably from about 0.035 mg/Kg to about 0.14 mg/Kg, and most preferably about 0.10 mg/Kg. In any case, a systemically sub-therapeutic amount is less than 1.0 mg/Kg.

The term "pharmaceutically acceptable acid addition salts" refers to salts of the subject compounds which possess the desired pharmacological activity and which are neither biologically nor otherwise undesirable. These salts are formed with inorganic acids such as hydrochloric acid, hydrobromic acid, sulfuric acid, nitric acid or phosphoric acid; or organic acids such as acetic acid, propionic acid, glycolic acid, pyruvic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid and the like.

The term "mammal" as used herein refers to domesticated mammals such as cattle, horses, swine, sheep, goats, dogs, cats, and the like.

All percentages used herein are "weight/weight" percentages (w/w).

The term "parenteral injection" as used herein refers to administration by subcutaneous or intramuscular injection.

One aspect of the invention is a composition suitable for parenteral injection for inducing estrus in a female mammal, or abortion or parturition in a pregnant mammal, which composition comprises an effective amount of a $PGF_2\alpha$ derivative, a systemically sub-therapeutic

amount of an antibiotic, and a pharmaceutically acceptable carrier.

Another aspect of the invention is the method for inducing estrus, abortion, or parturition in a female mammal, which method comprises administering a composition comprising an effective amount of a $PGF_2\alpha$ derivative, a systemically sub-therapeutic amount of an antibiotic, and a pharmaceutically acceptable carrier.

Another aspect of the invention is the method of administering a parenterally administerable compound without while preventing simultaneous infection, by including in the parenterally administered formulation a systemically sub-therapeutic amount of an antibiotic.

One aspect of the invention is a composition for parenteral administration to a mammal, which comprises a parenterally suitable compound, a systemically sub-therapeutic amount of an antibiotic, and a pharmaceutically acceptable carrier, especially where said parenterally suitable compound is a vaccine, a hormone, a vitamin, or nutritional supplement, particularly a prostaglandin or prostacyclin, or a derivative or analog thereof. A preferred sub-genus of the invention is a composition suitable for intramuscular administration to a female mammal, which composition comprises an effective amount of a $PGF_2\alpha$ derivative, a systemically sub-therapeutic amount of an antibiotic, and a pharmaceutically acceptable carrier, especially where said $PGF_2\alpha$ derivative is fenprostalene and said antibiotic is oxytetracycline. A preferred class of the invention is the composition wherein said fenprostalene is present in an amount between about 0.25 mg/mL and about 1.0 mg/mL, preferably about 0.5 mg/mL. A preferred sub-class of the invention is the composition in which

said oxytetracycline is present in an amount between about 0.4% and about 1.6%, preferably about 1.6%. A presently preferred embodiment of the invention is the composition comprising

| | |
|---|---|
| fenprostalene | 0.025% - 0.1% |
| oxytetracycline | 0.4% - 1.6% |
| dl-α-tocopherol | 0.025% - 0.050% |
| PEG 400 qs | 100.0%. |

Another aspect of the invention is a method for administering parenterally suitable compounds without injection-associated infection, which method comprises administering a composition comprising a parenterally suitable compound, a systemically sub-therapeutic amount of an antibiotic, and a pharmaceutically acceptable carrier. A preferred sub-genus of the invention is the method wherein said parenterally suitable compound is a vaccine, hormone, vitamin or nutritional supplement.

Another aspect of the invention is the method for inducing estrus, abortion, or parturition in a female mammal without initiating infection, which method comprises administering an effective amount of a $PGF_{2}\alpha$ derivative, a systemically sub-therapeutic amount of an antibiotic, and a pharmaceutically acceptable carrier, especially where said $PGF_{2}\alpha$ derivative is fenprostalene and said antibiotic is oxytetracycline. A preferred subgenus of the invention is the method wherein said fenprostalene is present in an amount between about 0.025% and about 0.050%, preferably about 0.050%. A preferred class of the invention is the method in which said oxytetracycline is present in an amount between about 0.4% and about 1.6%, preferably about 1.6%.

The systemically sub-therapeutic amount of antibiotic necessary to prevent injection-associated

infection may be determined by one of ordinary skill by routine experimentation. For example, one may inject guinea pigs with a composition containing a test amount of antibiotic and a lethal dose of bacteria to determine the amount of antibiotic necessary. The dose of bacteria used may be a combination of several strains, or may be a representative strain such as Clostridium chauveoi. The amount of antibiotic required will be independent of the species of subject mammal, but may be dependent on the species of bacteria. Thus, experiments with small laboratory animals are sufficient to establish dosages for larger animals. The use of a large excess (e.g., 10 times the lethal dose when administered without an antibiotic) of lethal bacteria is sufficient to compensate for any variation due to differences in bacterial species.

## ADMINISTRATION AND FORMULATION

One aspect of the present invention relates to pharmaceutical compositions useful for inducing estrus or parturition in female mammals, comprising an effective amount of a compound of $PGF_2\alpha$ derivative and a sub-therapeutic amount of an antibiotic, in admixture with a pharmaceutically acceptable non-toxic carrier. An effective amount of a $PGF_2\alpha$ derivative is that amount which is necessary to induce estrus in a female mammal, or the amount necessary to induce parturition in a pregnant mammal near the end of that mammal's gestation period.

Useful pharmaceutical carriers for the preparation of the pharmaceutical compositions hereof are liquids and can take the form of solutions, suspensions, elixirs, and the like. Carriers can be selected from the various oils, including those of petroleum, animal, vegetable or synthetic origin, for example, peanut oil, soybean oil,

mineral oil, sesame oil, and the like. Water, saline, aqueous dextrose, and glycols are preferred liquid carriers. Other suitable pharmaceutical carriers and their formulations are described in "Remington's Pharmaceutical Sciences" by E. W. Martin.

The compositions of the invention are prepared in solution form using standard techniques. $PGF_{2}\alpha$ derivatives are available from commercial sources, or may be prepared by the methods taught in the art. It is preferred to include a small amount of an anti-oxidizing agent such as dl-α-tocopherol in the formulation to protect the prostaglandin from oxidation. Antibiotics are also available from commercial sources.

The compositions of the invention may be assayed for efficacy by injecting suitable test animals with mixtures of compositions of the invention and lethal doses of infectious bacteria of a type commonly found on animal hide. For example, *Clostridium chauveoi* is a potentially lethal species of bacteria which may be used as a suitable challenge bacteria.

## EXAMPLE 1

(Formulations)

A formulation suitable for subcutaneous injection for inducing abortion, parturition, or estrus in cattle is prepared as follows:

| | | |
|---|---|---|
| fenprostalene | | 0.5 mg |
| oxytetracycline | | 16.0 mg |
| dl-α-tocopherol | | 0.5 mg |
| sterile polyethylene glycol 400 | qs | 1.0 mL |

The fenprostalene and oxytetracycline are added to a solution of dl-α-tocopherol and sterile polyethylene glycol 400, and the resulting solution is mixed well.

0234481

## EXAMPLE 2

(Formulations)

Other formulations suitable for intramuscular injection in cattle were prepared as follows:

(A) Progesterone

| | | |
|---|---|---|
| progesterone | | 100.0 mg |
| oxytetracycline | | 16.0 mg |
| sterile water for injection | qs | 5.0 mL |

(B) Oxytocin

| | |
|---|---|
| purified oxytocin principle (10 U/mL) | 1.5 mL |
| oxytetracycline | 16.0 mg |
| sterile water for injection | 1.0 mL |

(C) Cortisone acetate:

| | | |
|---|---|---|
| cortisone acetate | | 1.5 g |
| oxytetracycline | | 16.0 mg |
| sterile water for injection | qs | 5.0 mL |

## EXAMPLE 3

(Guinea Pig Assay)

Nine groups of five Guinea pigs (587-720 g/animal, Biolabs) were selected at random for challenge with Clostridium chauveoi. A 0.5 mL (100 $LD_{50}$ per 0.5 mL dose) dose of the indicated formulation (containing PEG 400, dl-α-tocopherol, oxytetracycline, and C. chauveoi) was administered by intramuscular injection to the left rear leg of each animal:

| Group# | Formulation *C. chauveoi* (per mL) | PEG 400 (for 1 mL) | tocopherol (mg/mL) | oxytetracycline (mg/mL) |
|---|---|---|---|---|
| 1 | 200 $LD_{50}$ | qs | 0.25 | 0.0 |
| 2 | 200 $LD_{50}$ | qs | 0.25 | 8.0 |
| 3 | 200 $LD_{50}$ | qs | 0.25 | 4.0 |
| 4 | 200 $LD_{50}$ | qs | 0.25 | 2.0 |
| 5 | 200 $LD_{50}$ | qs | 0.25 | 1.0 |
| 6 | 200 $LD_{50}$ | qs | 0.25 | 0.5 |
| 7 | 200 $LD_{50}$ | qs | 0.25 | 0.25 |
| 8 | 200 $LD_{50}$ | qs | 0.25 | 0.125 |
| 9 | 200 $LD_{50}$ | qs | (5% $CaCl_2$) | |

The number of animals surviving at 0, 18, 25, 42, 48, 72, and 96 hours post administration was counted and recorded. The results demonstrated that a systemically sub-therapeutic amount of oxytetracycline was effective to prevent injection-associated infection by *C. chauveoi*.

CLAIMS:

1. A composition suitable for parenteral injection in a bird or mammal, which composition comprises:

an effective amount of a parenterally suitable compound;

a systemically sub-effective amount of an antibiotic;

and

a pharmaceutically acceptable carrier.

2. The composition of claim 1, wherein said parenterally suitable compound is a vaccine, hormone, vitamin or nutritional supplement.

3. A composition suitable for parenteral injection for inducing estrus in a female mammal, or abortion or parturition in a pregnant mammal, which composition comprises:

an effective amount of a $PGF_2\alpha$ derivative;

a systemically sub-effective amount of an antibiotic;

and

a pharmaceutically acceptable carrier.

4. The composition of claim 3, wherein said $PGF_2\alpha$ derivative is fenprostalene and said antibiotic is oxytetracycline.

5. The composition of claim 4, wherein said fenprostalene is present in an amount between about 0.25 mg/mL and about 1.0 mg/mL.

6. The composition of claim 4, wherein said oxytetracycline is present in an amount between about 0.4% and about 1.6%.

7. The composition of claim 4, which comprises:

0.025% - 0.050% fenprostalene;

0.4 - 1.6% oxytetracycline; and

a pharmaceutically acceptable carrier.

8. The composition of claim 7, which comprises:
0.025% - 0.050% fenprostalene;
0.25% - 0.50% dl-α-tocopherol;
0.4% - 1.6% oxytetracycline; and
a pharmaceutically acceptable carrier.

9. The use of a composition according to claim 1 or 2 for the manufacture of a medicament for administering a parenterally suitable compound to a bird or mammal without initiating infection.

10. The use of a composition according to any one of claims 3 to 8 for the manufacture of a medicament inducing estrus, abortion or parturition in a female mammal without initiating infection.

European Patent Office

# EUROPEAN SEARCH REPORT

0234481
Application number

EP 87 10 2214

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US-A-3 985 791 (JOSEPH M. MUCHOWSKI)<br>----- | 1-10 | A 61 K 45/06<br>A 61 K 31/65<br>A 61 K 31/557//<br>(A 61 K 31/65<br>A 61 K 31:557) |
| | | | **TECHNICAL FIELDS SEARCHED (Int. Cl.4)** |
| | | | A 61 K |

The present search report has been drawn up for all claims

| Place of search | Date of completion of the search | Examiner |
|---|---|---|
| THE HAGUE | 21-05-1987 | BRINKMANN C. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82